# FASCICULE DE BREVET EUROPEEN

(11) **EP 0 900 084 B1**
(45) Date de publication et mention de la délivrance du brevet: **30.03.2005**
(21) Numéro de dépôt: 97923146.1
(22) Date de dépôt: 06.05.1997
(51) Int. Cl.: A61K 31/535, A61K 31/5375, A61P 1/00, A61P 35/00

(54) **UTILISATION DU BROMURE DE PINAVERIUM DANS LA PREVENTION DES PHENOMENES PROLIFERATIFS DES CELLULES DU TRACTUS HEPATODIGESTIF ET DES MALADIES QUI EN RESULTENT**
VERWENDUNG VON PINAVERIUM BROMID ZUR VORBEUGUNG DER VERMEHRUNG DER ZELLEN DES HEPATO-VERDAUUNGSTRAKTES UND DARAUS RESULTIERENDE ERKRANKUNGEN
USE OF PANAVERIUM BROMIDE FOR PREVENTING CELL PROLIFERATION AND DISEASES CAUSED THEREBY IN THE LIVER AND DIGESTIVE TRACT

(30) Priorité: 06.05.1996 FR 9605640
(43) Date de publication de la demande: 10.03.1999
(73) Titulaire: Solvay Pharma SAS, 92150 Suresnes (FR)
(72) Inventeur: CHRISTEN, Marie-Odile, F-75116 Paris Cedex (FR); MAUGARD, Joelle, F-78160 Marly le Roi (FR); DUC, Huynh, F-94800 Villejuif (FR); SCHERÜBL, Hans, D-12161 Berlin (DE); HUIZINGA, Jan, Puslinch, Ontario N0B 2J0 (CA); BLENNERHASSET, Michael, G., Toronto, Ontario M6R 2G2 (CA)
(74) Mandataire: Bernasconi, Jean Raymond
(86) Numéro de dépôt international: PCT/FR1997/000809
(87) Numéro de publication internationale: WO 1997/041859

(56) Documents cités:
- EP-A- 0 406 743
- FR-A- 2 097 032
- GASTROENTEROLOGY, vol. 112, no. 4, Avril 1997, page A731 XP002039802 J. FIORAMONTI ET AL.: "EFFECT OF A SELECTIVE CALCIUM ANTAGONIST, PINAVERIUM BROMIDE, ON SENSITIVITY TO RECTAL DISTENTION IN EXPERIMENTAL INFLAMMATION IN RATS"
- GASTROENTEROLOGY, vol. 112, no. 4, Avril 1997, page A703 XP002039803 J.C. BOYER ET AL.: "DIFFERENTIAL SENSITIVITY OF ISOLATED SMOOTH MUSCLE CELLS FROM NORMAL AND INFLAMED HUMAN COLONS TO CONTRACTILE AGENTS.EFFECTS OF CALCIUM CHANNEL BLOCKERS"
- GASTROENTEROLOGY, vol. 110, no. 4, Avril 1996, page A589 XP002039804 H. SCHERÜBL ET AL.: "ANTIPROLIFERATIVE EFFECTS OF CALCIUM CHANNEL BLOCKERS IN CARCINOID TUMOR CELLS"
- BORDEAUX MEDICAL, vol. 10, no. 21, 1977, pages 1457-1459, XP000614156 J.-J. DUBARRY ET AL.: "EFFET A COURT TERM DU BROMURE DE PINAVERIUM DANS LES OESOPHAGITES, GASTRO-DUODENITES ET COLOPATHIES FONCTIONNELLES"
- DRUGS TODAY, vol. 13, no. 6, 1978, pages 241-243, XP000614119 NEUMAN M.: "PINAVERIUM BROMIDE"
- GEN. PHARMAC., vol. 21, no. 6, 1990, pages 821-825, XP000614072 M.O. CHRITEN: "ACTION OF PINAVERIUM BROMIDE, A CALCIUM-ANTAGONIST, ON GASTROINTESTINAL MOTILITY DISORDERS"
- GASTROENTEROLOGY, vol. 104, no. S4, 1995, page A912 XP000614044 B.A. SCHEINFELD ET AL.: "VERAPAMIL SELECTIVELY INHIBITS INFLAMMATION-INDUCED INTESTINAL SMOOTH MUSCLE HYPERPLASIA IN VIVO: A POTENTIAL NEW THERAPY FOR STRICTURE FORMATION"
- PANCREAS, vol. 9, no. 2, 1994, pages 193-202, XP000614048 K. SATO ET AL.: "INHIBITORY EFFECT OF CALCIUM CHANNEL BLOCKERS ON GROWTH OF PANCREATIC CANCER CELLS"
- CANCER INVESTIGATION, vol. 8, no. 5, 1990, pages 451-458, XP000614047 L.R. ZACHARSKI ET AL.: "CHRONIC CALCIUM ANTAGONIST USE IN CARCINOMA OF THE LUNG AND COLON: A RETROSPECTIVE COHORT OBSERVATIONAL STUDY"
- ACTA GASTROENTEROLOGICA LATINOAMERICANA, vol. 25, no. 3, 1995, pages 137-144, XP000614034 D. AWAD ET AL.: "IRRITABLE BOWEL SYNDROME TREATMENT USING PINAVERIUM BROMIDE AS A CALCIUM CHANNEL BLOCKER"
- MEDECINE INTERNE, vol. 15, no. 12, 1980, pages 445-446, XP000614032 M. HALFON: "LE TRAITEMENT DES COLOPATHIES FONCTIONNELLES PAR LE BROMURE DE PINAVERIUM"

## Description

La présente invention est relative à une nouvelle utilisation du bromure de Pinaverium dans les pathologies gastrointestinales, maladies inflammatoires digestives (IBD), colites, ulcères, maladie de Crohn ou colopathies fonctionnelles (IBS), au cours desquelles apparaissent des phénomènes prolifératifs entraînant des complications pathologiques.

Le renouvellement constant et rapide de l'épithélium du tractus digestif est un élément important pour le maintien de l'intégrité de la muqueuse mais il l'est aussi pour le développement de tumeurs et d'hypertrophies.

On observe toujours dans ces derniers cas une prolifération anormale des cellules épithéliales et un nombre accru des zones prolifératives ; ceci se retrouve également chez les patients à risques familiaux de cancer du colon, ou chez les patients souffrant de maladies prédisposant au cancer du tube digestif (gastrites chroniques, maladies inflammatoires digestives, maladie de Crohn, colites ulcératives, polypes coloniques...). Par ailleurs, il a été montré que l'inflammation de l'intestin peut provoquer à la fois une hypertrophie des cellules musculaires lisses, et une hyperplasie. Il en résulte un épaississement de la paroi musculaire et donc une altération de la motilité, entraînant l'apparition de complications nécessitant des traitements chirurgicaux. Le Verapamil a été proposé pour inhiber l'hyperplasie des cellules musculaires lisses intestinales, induite par une inflammation (B.A. Scheinfeld et M. G. Blennerhassett, 1995, Gastroenterology, vol 108, No. 4).

De plus, l'inhibition de la prolifération des cellules cancéreuses est un facteur déterminant pour la prévention et le traitement des cancers du tube digestif ainsi que des métastases.

Le bromure de Pinaverium est le seul antagoniste calcique sélectif du tractus' intestinal, à activité spasmolytique qui est prescrit en traitement chronique des troubles fonctionnels digestifs ("Irritable Bowel Syndrome" ou IBS). Ce composé a notamment été décrit dans le brevet français 2 097 032.

Dubarry et Quiton (Bordeaux Medical, 1977, Vol. 10(21)) ont montré que le bromure de Pinaverium avait des propriétés antispasmodiques. Il a été aussi rapporté (Drugs of Today, 1977, Vol. XIII(6)) que le bromure de Pinaverium était un agent spasmolytique approprié pour le traitement de l'ulcère gastrique et du duodéum, des colites ou des dyskinésies biliaires ou d'oesophage. Enfin, Scherübl H. et al. (1996, Gastroenterology, Vol 110(4)) ont rapporté l'activité inhibitrice du bromure de Pinaverium vis-à-vis de la prolifération d'une lignée de cellules pancréatique carcinoïdes.

De façon surprenante, les auteurs de la présente invention ont maintenant montré par des études réalisées in vitro que le bromure de Pinaverium est capable d'inhiber la prolifération de cellules cancéreuses et hyperplasiques du tube digestif et ce, de façon supérieure à un autre antagoniste calcique, le Verapamil. Par ailleurs, une étude réalisée in vivo a permis de montrer également l'efficacité du bromure de Pinaverium sur la diminution de tumeurs induites chez l'animal.

Le bromure de Pinaverium peut donc être utilisé en tant que médicament préventif d'un cancer du tube digestif chez le sujet sain comme chez le sujet atteint de pathologie digestive, tel que l'IBS. Il exercera alors chez ce dernier un effet préventif ou curatif complémentaire de son effet sur l'IBS.

Ce composé peut également être utilisé en traitement curatif de l'hépatocarcinome ou de cancer du tractus hépatodigestif.

La présente invention a donc pour objet l'utilisation du bromure de Pinaverium pour la préparation d'un médicament destiné à la prévention ou le traitement des maladies du tractus hépatodigestif dues à une prolifération excessive des cellules de celui-ci.

Plus particulièrement, l'invention a pour objet l'utilisation du bromure de Pinaverium pour la préparation d'un médicament destiné à la prévention ou le traitement des hyperplasies cellulaires intestinales liées aux maladies inflammatoires digestives, ou à la prévention des hypertrophies de la paroi des cellules musculaires lisses du tractus intestinal et des troubles de la motricité dues aux maladies inflammatoires du tube digestif ("Inflammatory Bowel Disease" ou IBD, maladie de Crohn, colite ulcérative) caractéristiques de l'IBS.

L'invention vise également l'utilisation du bromure de Pinaverium pour la préparation d'un médicament destiné à la prévention ou au traitement d'un cancer du tractus hépatodigestif comprenant le cancer du colon et de l'estomac, l'hépatocarcinome.

Elle a également pour objet l'utilisation du bromure de Pinaverium pour la préparation d'un médicament destiné à la prévention et au traitement du cancer du tractus hépatodigestif chez des patients souffrant de syndromes du colon irritable ("Irritable Bowel Syndrome" ou IBS), en traitement complémentaire et simultané de celui des symptômes caractéristiques de l'IBS.

Elle vise enfin l'utilisation du bromure de Pinaverium pour la fabrication d'un médicament destiné au traitement des maladies inflammatoires digestives telles que l'IBD (maladie inflammatoire intestinale ou "Inflammatory Bowel Disease"), la maladie de Crohn ou la colite ulcérative, généralement associées à l'épaississement de la muqueuse.

Préférentiellement, dans le cadre de la présente invention, le bromure de Pinaverium est utilisé en étant administré en une quantité journalière comprise entre 50 mg à 450 mg. L'administration est réalisée par voie orale, ou par voie injectable, à partir d'une solution dosée à 0,2 à 1 mg/ml.

Les avantages de l'invention apparaissent de façon plus détaillée dans les essais décrits ci-après.

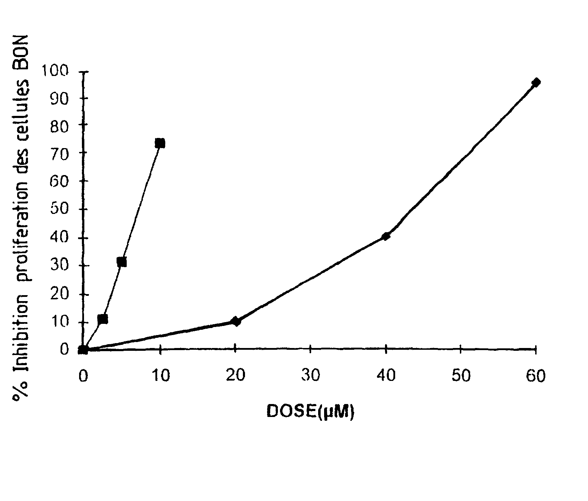

Par ailleurs, la figure suivante montre l'effet du bromure de Pinaverium (_■_) sur l'inhibition de la croissance de cellules carcinoïdes pancréatiques comparé à celui du Verapamil (-◇-).

### Essai 1 :

### Inhibition de la prolifération des cellules du cancer du colon par le bromure de Pinaverium comparée à celle induite par un autre inhibiteur calcique (Verapamil)

La lignée cellulaire de cancer du colon HT29 utilisée (à l'origine isolée à partir d'adénocarcinome humain du colon) a été obtenue auprès de l'ATCC (American Type Culture Collection).

Les cellules sont cultivées à raison de 10⁴ ou 5 x 10⁴ cellules dans un puits de 200 µl de milieu de culture composé de RPMI 1640 supplémenté de 10 % de sérum de veau foetal (FCS), 2 mM de I-glutamine et 25 µM de gentamicine. Le bromure de Pinaverium (ou le Verapamil), mis en solution dans l'éthanol à 50 % ou le DMSO, est ajouté à la culture de cellule à diverses concentrations, et le pourcentage d'inhibition de la prolifération cellulaire est évalué par la mesure de l'incorporation de la thymidine tritiée (³HThdR) ; celle-ci étant ajoutée dans chaque puits à raison de 37 KBq (1 µCi), les puits sont prélevés et comptés 15 heures plus tard au moyen de "Filter Mate™ Cell Harvester et Matrix 9600™ Direct Beta Counter" (Packard).

Les résultats sont les suivants :

| | Concentration du produit testé (% en poids) | % Inhibition bromure de Pinaverium | % Inhibition Verapamil |
|---|---|---|---|
| ³HThdR | 0,025 | 78 | 17 |
| | 0,05 | 82 | 17 |
| | 0,1 | 86 | 27 |

L'action inhibitrice du bromure de Pinaverium est de 3 à 5 fois supérieure à celle du Verapamil.

### Essai 2 :

### Action du bromure de Pinaverium sur les cellules d'hépatocarcinome

Les cellules d'hépatocarcinome de rat LFC (VPR42, Villejuif) sont cultivées à raison de 10⁴ ou 5 x 10⁴ cellules dans un puits de 200 µl de milieu de culture. Les produits à tester (bromure de Pinaverium et Verapamil) sont mis en solution dans l'éthanol à 50% et utilisés aux concentrations de 0,1% et 1% en poids.

L'inhibition de la prolifération cellulaire est évaluée à l'aide de thymidine tritiée tel que décrit dans l'essai 1. L'évaluation est faite comparativement au produit de référence qui est le Verapamil. Les résultats montrent un pourcentage d'inhibition de prolifération de 85 à 88% pour le bromure de Pinaverium et de 72% pour le Verapamil.

### Essai 3 :

### Effet du bromure de Pinaverium sur la croissance des cellules intestinales carcinoïdes STC-1

Les lignées de cellules de cancer intestinal de souris STC-1 sont cultivées dans un milieu DMEM contenant 15 % de sérum de cheval et 2,5 % FCS. Les produits à étudier sont ajoutés à la culture et la croissance cellulaire évaluée par comptage des cellules ou plus souvent par technique de fluorescence, basée sur l'hydrolyse enzymatique cellulaire du substrat fluorogenetic, le 4-méthylumbelliferyl heptanoate (MUH); en effet, les cellules vivantes incubées avec MUH génèrent un signal fluorescent proportionnel à leur nombre et déterminé à l'aide d'un "Titentec Fluoroscan II" (Flow Laboratory), et donné en unité arbitraire de fluorescence.

Le bromure de Pinaverium s'est montré dans le test deux fois plus actif que le Verapamil.
(DE₅₀ PINAVERIUM = 7 µM ; DE₅₀ VERAPAMIL = 13 µM)

### Essai 4 (de référence):

### Etude de l'inhibition de la croissance des cellules carcinoïdes pancréatiques BON par le bromure de Pinaverium

Les lignées de cellules humaines carcinoïdes pancréatiques BON sont cultivées dans un milieu DMEM / F12K (1/1) additionné de 10 % FCS. Le Verapamil et le bromure de Pinaverium sont ajoutés à la culture et la croissance cellulaire est déterminée au bout de 8 jours par une technique de fluorescence telle que décrite dans l'essai 3.

Les résultats reportés sur la figure montrent l'efficacité du bromure de Pinaverium dans l'inhibition de la croissance des cellules carcinoïdes pancréatiques BON, et sa supériorité sur le Verapamil
(DE₅₀ PINAVERIUM = 7,2 µM ; DE₅₀ VERAPAMIL + 43,6 µM) .

### Essai 5 :

### Inhibition par le bromure de Pinaverium de l'hyperplasie des cellules du muscle lisse intestinal de rat induite par le PDGF (Platelet Derived Growth Factor)

Les cellules musculaires lisses intestinales sont cultivées dans un milieu de culture contenant 10 % de sérum foetal bovin, puis sont exposées au PDGF (facteur de croissance retrouvé au cours des inflammations) pendant 20 heures. La croissance cellulaire est évaluée par l'incorporation de thymidine tritiée.

Le bromure de Pinaverium (10⁻⁵ M) inhibe totalement la croissance des cellules musculaires lisses intestinales induites par le facteur d'inflammation PDGF. Il a donc un effet bénéfique sur les phénomènes d'inflammation du tube digestif.

### Essai 6 :

### Effet du bromure de Pinaverium sur le diamètre des tumeurs induites chez la souris " nude "

Les tumeurs sont induites par injection sous-cutanée de cellules de cancer du colon HT29 chez des souris " nude ".

Le bromure de Pinaverium est injecté par voie intra-péritoneale quelques heures avant l'injection des cellules HT29.

L'évolution tumorale est évaluée par la mesure du diamètre de la tumeur locale induite en fonction de la durée de l'expérimentation qui est de 44 jours.

On observe une réduction du diamètre des tumeurs de 30 % à partir du 25ème jour de l'expérimentation.

## Revendications

1. Utilisation du bromure de Pinaverium pour la préparation d'un médicament destiné à la prévention ou au traitement d'une prolifération excessive des cellules du tractus hépatodigestif que sont les cellules de l'estomac, des intestins, du colon ou du foie.

2. Utilisation selon la revendication 1, dans laquelle la prolifération cellulaire excessive est une hyperplasie cellulaire intestinale ou une hypertrophie de la paroi des cellules musculaires lisses du tractus intestinal.

3. Utilisation selon la revendication 1 pour la préparation d'un médicament destiné à la prévention ou au traitement d'un cancer du tractus hépatodigestif comprenant le cancer du colon, de l'estomac et l'hépatocarcinome.

4. Utilisation selon la revendication 1 pour la préparation d'un médicament destiné à la prévention et au traitement du cancer du tractus hépatodigestif chez des patients souffrant de syndromes du colon irritable ("Irritable Bowel Syndrom" ou IBS), en traitement complémentaire et simultané de celui des symptômes caractéristiques de l'IBS.

5. Utilisation du bromure de Pinaverium pour la fabrication d'un médicament destiné au traitement d'un épaississement de la muqueuse du tube digestif associé à des maladies inflammatoires digestives telles que la maladie inflammatoire intestinale ("Inflammotory Bowel disease" ou IBD), la maladie de Crohn ou la colite ulcérative.

6. Utilisation selon l'une des revendications 1 à 5, dans laquelle le bromure de Pinaverium est utilisé en quantité suffisante pour être administré à raison de 50 à 450 mg par jour par voie orale, ou bien par voie injectable, à partir d'une solution dosée à 0,2 à 1 mg/ml.

## Patentansprüche

1. Verwendung von Pinaveriumbromid für die Zubereitung eines Medikaments zur Vorbeugung oder zur Behandlung einer übermäßigen Vermehrung der Zellen des hepatodigestiven Trakts, und zwar der Zellen des Magens, der Därme, des Kolon oder der Leber.

2. Verwendung nach Anspruch 1, bei der die übermäßige Zellvermehrung eine Darmzellenhyperplasie oder eine Hypertrophie der Zellwände der glatten Muskulatur des Darmtrakts ist.

3. Verwendung nach Anspruch 1 für die Zubereitung eines Medikaments zur Vorbeugung oder zur Behandlung von Krebs des hepatodigestiven Trakts, umfassend Kolonkrebs, Magenkrebs und Hepatokarzinom.

4. Verwendung nach Anspruch 1 für die Zubereitung eines Medikament zur Vorbeugung und zur Behandlung von Krebs des hepatodigestiven Trakts bei Patienten, die an Reizkolonsyndromen ("Irritable Bowel Syndrom" oder IBS) leiden, zur ergänzenden und gleichzeitigen Behandlung zu der der charakteristischen Symptome des IBS.

5. Verwendung von Pinaveriumbromid für die Herstellung eines Medikaments zur Behandlung einer Verdickung der Schleimhaut des Verdauungstrakts in Verbindung mit entzündlichen Verdauungskrankheiten, wie entzündliche Darmkrankheit ("Inflammatory Bowel disease" oder IBD), Crohn-Krankheit oder Kolitis ulzerosa.

6. Verwendung nach einem der Ansprüche 1 bis 5, bei der das Pinaveriumbromid in einer ausreichenden Menge verwendet wird, um ausgehend von einer mit 0,2 bis 1 mg/ml dosierten Lösung in einer Menge von 50 bis 450 mg täglich oral oder durch Injektion verabreicht zu werden.

## Claims

1. Use of pinaverium bromide for the preparation of a medication intended for the prevention or treatment of an excessive proliferation of cells of the hepatodigestive tract, that are cells of the stomach, intestines, colon or liver.

2. Use according to claim 1, wherein the excessive cellular proliferation is intestinal cellular hyperplasia or hypertrophia of the wall of the smooth muscular cells of the intestinal tract.

3. Use according to claim 1 for the preparation of a medication intended for the prevention or treatment of cancer of the hepatodigestive tract comprising cancer of the colon and stomach, and hepatocarcinoma.

4. Use according to claim 1 for the preparation of a medication intended for the prevention or treatment of cancer of the hepatodigestive tract in patients suffering from irritable bowel syndrome or IBS and in the simultaneous complementary treatment of symptoms characteristic of IBS.

5. Use of pinaverium bromide for the production of a medication intended for the treatment of a thickening of the mucosae of the digestive tube associated with inflammatory digestive diseases such as inflammatory bowel disease or IBD, Crohn's disease or ulcerative colitis.

6. Use according to one of claims 1 to 5, wherein pinaverium bromide is used in a sufficient quantity to be administered at the rate of 50 to 450 mg per day, orally or indeed by injection, with a solution containing 0.2 to 1 mg/ml.
